# EUROPEAN PATENT SPECIFICATION

(11) **EP 4 478 962 B1**
(45) Date of publication and mention of the grant of the patent: **17.06.2026**
(21) Application number: 23716955.2
(22) Date of filing: 17.03.2023
(51) Int. Cl.: A61B 8/12, A61B 8/00, A61B 5/00, A61B 8/08

(54) **INTRAVASCULAR IMAGING DEVICES WITH A WIRELESS ROTARY MOTOR**
INTRAVASKULÄRE BILDGEBUNGSVORRICHTUNGEN MIT EINEM DRAHTLOSEN DREHMOTOR
DISPOSITIFS D'IMAGERIE INTRAVASCULAIRE AVEC MOTEUR ROTATIF SANS FIL

(30) Priority: 17.03.2022 US 202263320810 P
(43) Date of publication of application: 25.12.2024
(73) Proprietor: Boston Scientific Scimed, Inc., Maple Grove, MN 55311 (US)
(72) Inventor: FLANAGAN, Aiden, Kilcolgan, H91FV1X (IE); FAWDRY, Martin, Galway, H91K5Y4 (IE)
(74) Representative: Vossius & Partner Patentanwälte Rechtsanwälte mbB
(86) International application number: PCT/US2023/015502
(87) International publication number: WO 2023/177868

(56) References cited:
- CN-A- 109 044 404
- US-A1- 2005 143 664
- US-A1- 2010 249 603
- US-B2- 8 147 414

## Description

### Cross-Reference To Related Applications

This application claims the benefit of priority of U.S. Provisional Application No. 63/320,810, filed March 17, 2022.

### Technical Field

The present disclosure pertains to medical imaging. More particularly, the present disclosure pertains to intravascular imaging.

### Background

A wide variety of medical devices have been developed for medical use, for example, intravascular use. Some of these devices include intravascular imaging devices. In addition, methods for intravascular imaging have been developed. Of these devices and methods, each has certain advantages and disadvantages. There is an ongoing need to provide alternative devices as well as alternative methods.

US 2010/249603 A1 describes an intravascular device according to the preamble of claim 1.

US 2005/143664 A1 describes a scanning probe using MEMS micromotor for endosocopic imaging, where a movable scanner is coupled to the motor, so that the scanner is directed towards a transmission path.

CN 109 044 404 A discloses an ultrasound catheter comprising a wireless power receiving module, configured to receive the wireless power transmitted by the wireless power transmitting module of an ultrasonic controller.

### Brief Summary

The object of this invention is achieved with an intravascular device according to claim 1. Preferred embodiments are defined in the dependent claims. This disclosure provides design, material, manufacturing method, and use alternatives for medical devices. An intravascular imaging device is disclosed. The intravascular imaging device comprises: a catheter shaft; an imaging core disposed within the catheter shaft, the imaging core including a sheath, an imaging emitter/transducer secured relative to the sheath, a reflector, and a wireless rotary motor coupled to the reflector; an antenna disposed adjacent to the imaging emitter/transducer; and a rectenna disposed adjacent to the wireless rotary motor.

Alternatively or additionally to any of the embodiments above, the imaging emitter/transducer includes an ultrasound transducer.

Alternatively or additionally to any of the embodiments above, the imaging emitter/transducer includes an optical coherence tomography emitter.

Alternatively or additionally to any of the embodiments above, the imaging emitter/transducer includes both an ultrasound transducer and an optical coherence tomography emitter.

Alternatively or additionally to any of the embodiments above, the rectenna is disposed adjacent to a distal end region of the wireless rotary motor.

Alternatively or additionally to any of the embodiments above, the rectenna is disposed adjacent to a proximal end region of the wireless rotary motor.

Alternatively or additionally to any of the embodiments above, the reflector includes an angled surface.

Alternatively or additionally to any of the embodiments above, the reflector includes a curved surface.

Alternatively or additionally to any of the embodiments above, the reflector includes an annular surface.

Alternatively or additionally to any of the embodiments above, a guidewire lumen is formed in the imaging core.

Alternatively or additionally to any of the embodiments above, the guidewire lumen extends through the imaging emitter/transducer, the wireless rotary motor, or both.

Alternatively or additionally to any of the embodiments above, the imaging emitter/transducer is axially fixed relative to the sheath.

Alternatively or additionally to any of the embodiments above, the imaging emitter/transducer is rotationally fixed relative to the sheath.

An intravascular imaging device is disclosed. The intravascular imaging device comprises: a catheter shaft; an imaging core disposed within the catheter shaft, the imaging core including a sheath, an ultrasound transducer rotationally fixed relative to the sheath, a reflector, and a wireless rotary motor coupled to the reflector; wherein the wireless rotary motor is configured to rotate the reflector relative to the ultrasound transducer; a transmitter disposed adjacent to the ultrasound transducer; a receiver disposed adjacent to the wireless rotary motor; and wherein the transmitter is configured to transmit power to the receiver in order to power the wireless rotary motor.

Alternatively or additionally to any of the embodiments above, the reflector includes an angled surface.

Alternatively or additionally to any of the embodiments above, the reflector includes a curved surface.

Alternatively or additionally to any of the embodiments above, the reflector includes an annular surface.

Alternatively or additionally to any of the embodiments above, a guidewire lumen is formed in the imaging core.

Alternatively or additionally to any of the embodiments above, the guidewire lumen extends through the imaging emitter/transducer, the wireless rotary motor, or both.

A method for imaging is disclosed. The method comprises: disposing an intravascular imaging device within a blood vessel, the intravascular imaging device comprising: a catheter shaft, an imaging core disposed within the catheter shaft, the imaging core including a sheath, an imaging emitter/transducer secured relative to the sheath, a reflector, and a wireless rotary motor coupled to the reflector, an antenna disposed adjacent to the imaging emitter/transducer, and a rectenna disposed adjacent to the wireless rotary motor; activating the imaging emitter/transducer; and rotating the reflector with the wireless rotatory motor.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 schematically depicts an example intravascular imaging system.
FIG. 2 is a perspective view of an example intravascular imaging catheter system.
FIG. 3 is a side view of a portion of an example intravascular imaging catheter system.
FIG. 4 is a side view of a portion of an example intravascular imaging catheter system.
FIG. 5 is a side view of a portion of an example intravascular imaging catheter system.
FIG. 6 is a side view of a portion of an example intravascular imaging catheter system.
FIG. 7 is a side view of a portion of an example intravascular imaging catheter system.
FIG. 8 is a side view of a portion of an example intravascular imaging catheter system.
FIG. 9 is a side view of a portion of an example intravascular imaging catheter system.

While the disclosure is amenable to various modifications and alternative forms, specifics thereof have been shown by way of example in the drawings and will be described in detail. It should be understood, however, that the intention is not to limit the invention to the particular embodiments described. The scope of the invention is defined by the appended claims.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

Intravascular imaging devices insertable into patients have proven diagnostic capabilities for a variety of diseases and disorders. For example, intravascular ultrasound ("IVUS") imaging systems and or optical coherence tomography ("OCT") imaging systems may be used as an imaging modality for diagnosing blocked blood vessels and providing information to aid medical practitioners in selecting and placing stents and other devices to restore or increase blood flow. IVUS/OCT imaging systems may also be used to diagnose atheromatous plaque build-up at particular locations within blood vessels. IVUS/OCT imaging systems may also be used to determine the existence of an intravascular obstruction or stenosis, as well as the nature and degree of the obstruction or stenosis. IVUS/OCT imaging systems may also be used to visualize segments of a vascular system that may be difficult to visualize using other intravascular imaging techniques, such as angiography, due to, for example, movement (e.g., a beating heart) or obstruction by one or more structures (e.g., one or more blood vessels not desired to be imaged). IVUS/OCT imaging systems may also be used to monitor or assess ongoing intravascular treatments, such as angiography and stent placement in real (or almost real) time. Moreover, IVUS/OCT imaging systems may be used to monitor one or more heart chambers.

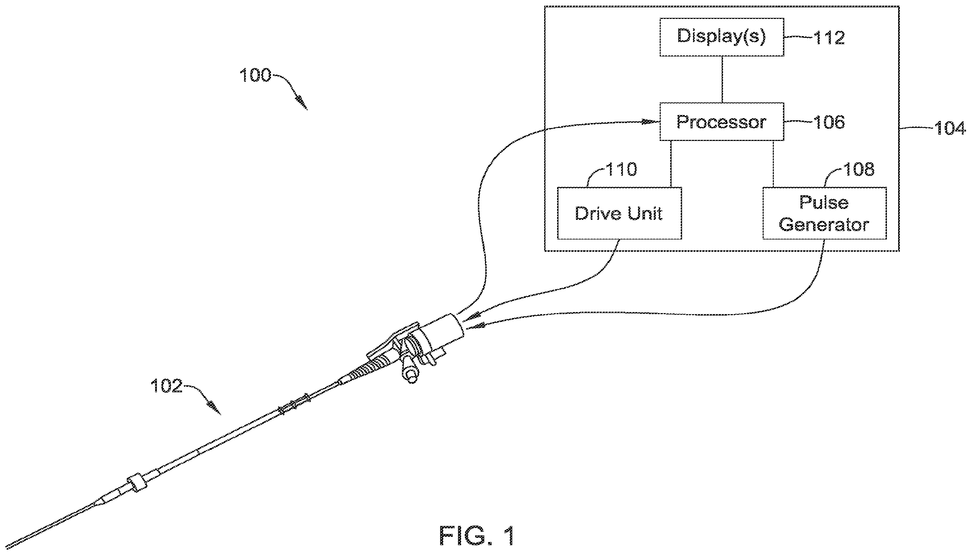

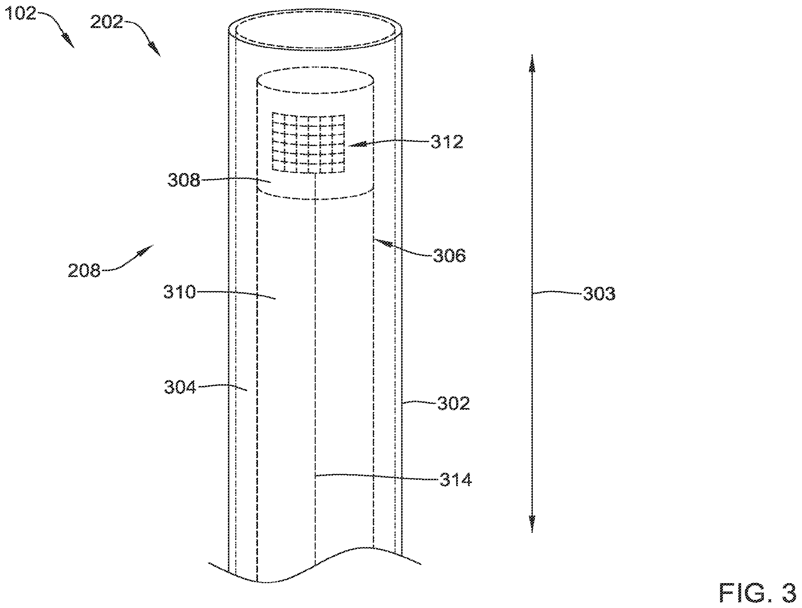

FIG. 1 illustrates schematically an example intravascular imaging system 100. In this example, the intravascular imaging system 100 takes the form of an IVUS imaging system. However, other imaging systems are contemplated including optical coherence tomography imaging systems. The IVUS imaging system 100 includes a catheter 102 that is coupleable to a processing unit or control module 104. The control module 104 may include, for example, a processor 106, a pulse generator 108, a drive unit 110, and one or more displays or display units 112. In some instances, the pulse generator 108 forms electric pulses that may be input to one or more transducers (312 in FIG. 3) disposed in the catheter 102.

For the purposes of this disclosure, the term "display" may either refer to an electronic device (e.g., such as a monitor, etc.) used for the visual representation of data or to the visual representation of data itself. In other words, the term "display" may refer to a hardware device for displaying data or the term "display" may refer to the data displayed on the hardware device. In some cases, it may be helpful to use the phrase "display unit" when referring to the hardware component and the term "display" when referring to the visual/data component shown on the display unit. However, such terminology need not be strictly adhered to in this disclosure or in the art in general. One of skill in the art would be able to discern when the term "display" is referring to a hardware component and when the term "display" is referring to the visual/data component.

In some instances, mechanical energy from the drive unit 110 may be used to drive an imaging core (306 in FIG. 3) disposed in the catheter 102. In some instances, electric signals transmitted from the one or more transducers (312 in FIG. 3) may be input to the processor 106 for processing. In some instances, the processed electric signals from the one or more transducers (312 in FIG. 3) can be displayed as one or more images on the one or more display units 112. For example, a scan converter can be used to map scan line samples (e.g., radial scan line samples, or the like) to a two-dimensional Cartesian grid to display the one or more images on the one or more display units 112.

In some instances, the processor 106 may also be used to control the functioning of one or more of the other components of the control module 104. For example, the processor 106 may be used to control at least one of the frequency or duration of the electrical pulses transmitted from the pulse generator 108, the rotation rate of the imaging core (306 in FIG. 3) by the drive unit 110, the velocity or length of the pullback of the imaging core (306 in FIG. 3) by the drive unit 110, or one or more properties of one or more images formed on the one or more display units 112.

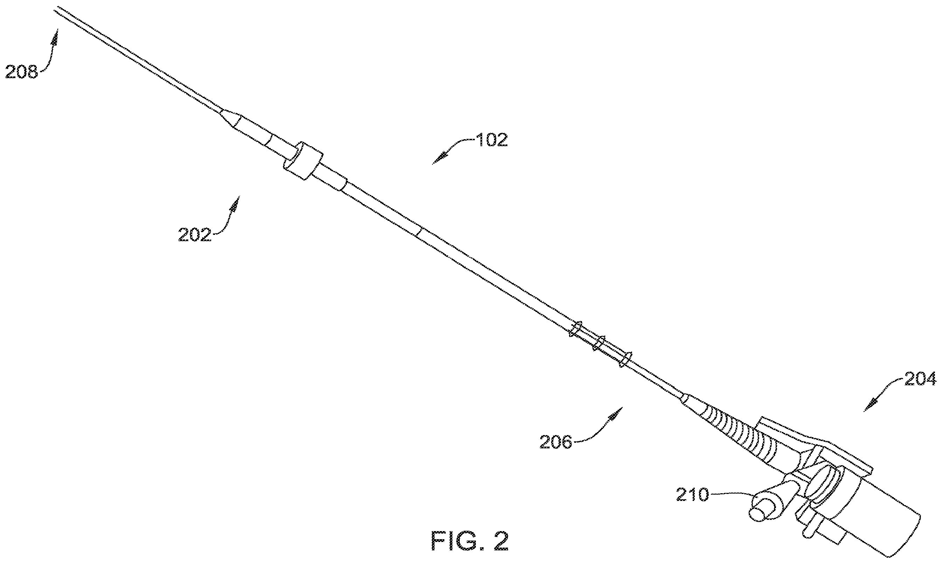

FIG. 2 is a schematic side view of one embodiment of the catheter 102 of the IVUS imaging system (100 in FIG. 1). The catheter 102 includes an elongated member 202 and a hub 204. The elongated member 202 includes a proximal end 206 and a distal end 208. In FIG. 2, the proximal end 206 of the elongated member 202 is coupled to the catheter hub 204 and the distal end 208 of the elongated member is configured and arranged for percutaneous insertion into a patient. Optionally, the catheter 102 may define at least one flush port, such as flush port 210. The flush port 210 may be defined in the hub 204. The hub 204 may be configured and arranged to couple to the control module (104 in FIG 1). In some instances, the elongated member 202 and the hub 204 are formed as a unitary body. In other instances, the elongated member 202 and the catheter hub 204 are formed separately and subsequently assembled together.

FIG. 3 is a schematic perspective view of one embodiment of the distal end 208 of the elongated member 202 of the catheter 102. The elongated member 202 includes a sheath 302 with a longitudinal axis 303 and a lumen 304. An imaging core 306 is disposed in the lumen 304. The imaging core 306 includes an imaging device 308 coupled to a distal end of a driveshaft 310 that is rotatable either manually or using a computer-controlled drive mechanism. One or more transducers 312 may be mounted to the imaging device 308 and employed to transmit and receive acoustic signals. The sheath 302 may be formed from any flexible, biocompatible material suitable for insertion into a patient. Examples of suitable materials include, for example, polyethylene, polyurethane, plastic, spiral-cut stainless steel, nitinol hypotube, and the like or combinations thereof.

In some instances, for example as shown in Figure 3, an array of transducers 312 are mounted to the imaging device 308. Alternatively, a single transducer may be employed. Any suitable number of transducers 312 can be used. For example, there can be two, three, four, five, six, seven, eight, nine, ten, twelve, fifteen, sixteen, twenty, twenty-five, fifty, one hundred, five hundred, one thousand, or more transducers. As will be recognized, other numbers of transducers may also be used. When a plurality of transducers 312 are employed, the transducers 312 can be configured into any suitable arrangement including, for example, an annular arrangement, a rectangular arrangement, or the like.

The one or more transducers 312 may be formed from materials capable of transforming applied electrical pulses to pressure distortions on the surface of the one or more transducers 312, and vice versa. Examples of suitable materials include piezoelectric ceramic materials, piezocomposite materials, piezoelectric plastics, barium titanates, lead zirconate titanates, lead metaniobates, polyvinylidene fluorides, and the like. Other transducer technologies include composite materials, single-crystal composites, and semiconductor devices (e.g., capacitive micromachined ultrasound transducers ("cMUT"), piezoelectric micromachined ultrasound transducers ("pMUT"), or the like).

The pressure distortions on the surface of the one or more transducers 312 form acoustic pulses of a frequency based on the resonant frequencies of the one or more transducers 312. The resonant frequencies of the one or more transducers 312 may be affected by the size, shape, and material used to form the one or more transducers 312. The one or more transducers 312 may be formed in any shape suitable for positioning within the catheter 102 and for propagating acoustic pulses of a desired frequency in one or more selected directions. For example, transducers may be disc-shaped, block-shaped, rectangular-shaped, oval-shaped, and the like. The one or more transducers may be formed in the desired shape by any process including, for example, dicing, dice and fill, machining, microfabrication, and the like.

As an example, each of the one or more transducers 312 may include a layer of piezoelectric material sandwiched between a matching layer and a conductive backing material formed from an acoustically absorbent material (e.g., an epoxy substrate with tungsten particles). During operation, the piezoelectric layer may be electrically excited to cause the emission of acoustic pulses.

The one or more transducers 312 can be used to form a radial cross-sectional image of a surrounding space. Thus, for example, when the one or more transducers 312 are disposed in the catheter 102 and inserted into a blood vessel of a patient, the one more transducers 312 may be used to form an image of the walls of the blood vessel and tissue surrounding the blood vessel.

The imaging core 306 is rotated about the longitudinal axis 303 of the catheter 102. As the imaging core 306 rotates, the one or more transducers 312 emit acoustic signals in different radial directions (e.g., along different radial scan lines). For example, the one or more transducers 312 can emit acoustic signals at regular (or irregular) increments, such as 256 radial scan lines per revolution, or the like. It will be understood that other numbers of radial scan lines can be emitted per revolution, instead.

When an emitted acoustic pulse with sufficient energy encounters one or more medium boundaries, such as one or more tissue boundaries, a portion of the emitted acoustic pulse is reflected back to the emitting transducer as an echo pulse. Each echo pulse that reaches a transducer with sufficient energy to be detected is transformed to an electrical signal in the receiving transducer. The one or more transformed electrical signals are transmitted to the control module (104 in FIG. 1) where the processor 106 processes the electrical-signal characteristics to form a displayable image of the imaged region based, at least in part, on a collection of information from each of the acoustic pulses transmitted and the echo pulses received. In some instances, the rotation of the imaging core 306 is driven by the drive unit 110 disposed in the control module (104 in FIG. 1). In alternate embodiments, the one or more transducers 312 are fixed in place and do not rotate. In which case, the driveshaft 310 may, instead, rotate a mirror that reflects acoustic signals to and from the fixed one or more transducers 312.

When the one or more transducers 312 are rotated about the longitudinal axis 303 of the catheter 102 emitting acoustic pulses, a plurality of images can be formed that collectively form a radial cross-sectional image (e.g., a tomographic image) of a portion of the region surrounding the one or more transducers 312, such as the walls of a blood vessel of interest and tissue surrounding the blood vessel. The radial cross-sectional image can, optionally, be displayed on one or more display units 112. The at least one of the imaging core 306 can be either manually rotated or rotated using a computer-controlled mechanism.

The imaging core 306 may also move longitudinally along the blood vessel within which the catheter 102 is inserted so that a plurality of cross-sectional images may be formed along a longitudinal length of the blood vessel. During an imaging procedure the one or more transducers 312 may be retracted (e.g., pulled back) along the longitudinal length of the catheter 102. The catheter 102 can include at least one telescoping section that can be retracted during pullback of the one or more transducers 312. In some instances, the drive unit 110 drives the pullback of the imaging core 306 within the catheter 102. The drive unit 110 pullback distance of the imaging core can be any suitable distance including, for example, at least 5 cm, 10 cm, 15 cm, 20 cm, 25 cm, or more. The entire catheter 102 can be retracted during an imaging procedure either with or without the imaging core 306 moving longitudinally independently of the catheter 102.

A stepper motor may, optionally, be used to pull back the imaging core 306. The stepper motor can pull back the imaging core 306 a short distance and stop long enough for the one or more transducers 306 to capture an image or series of images before pulling back the imaging core 306 another short distance and again capturing another image or series of images, and so on.

The quality of an image produced at different depths from the one or more transducers 312 may be affected by one or more factors including, for example, bandwidth, transducer focus, beam pattern, as well as the frequency of the acoustic pulse. The frequency of the acoustic pulse output from the one or more transducers 312 may also affect the penetration depth of the acoustic pulse output from the one or more transducers 312. In general, as the frequency of an acoustic pulse is lowered, the depth of the penetration of the acoustic pulse within patient tissue increases. In some instances, the IVUS imaging system 100 operates within a frequency range of 5 MHz to 100 MHz.

One or more conductors 314 can electrically couple the transducers 312 to the control module 104 (see, for example, FIG. 1). In which case, the one or more conductors 314 may extend along a longitudinal length of the rotatable driveshaft 310.

The catheter 102 with one or more transducers 312 mounted to the distal end 208 of the imaging core 308 may be inserted percutaneously into a patient via an accessible blood vessel, such as the femoral artery, femoral vein, or jugular vein, at a site remote from the selected portion of the selected region, such as a blood vessel, to be imaged. The catheter 102 may then be advanced through the blood vessels of the patient to the selected imaging site, such as a portion of a selected blood vessel.

An image or image frame ("frame") can be generated each time one or more acoustic signals are output to surrounding tissue and one or more corresponding echo signals are received by the imager 308 and transmitted to the processor 106. Alternatively, an image or image frame can be a composite of scan lines from a full or partial rotation of the imaging core or device. A plurality (e.g., a sequence) of frames may be acquired over time during any type of movement of the imaging device 308. For example, the frames can be acquired during rotation and pullback of the imaging device 308 along the target imaging location. It will be understood that frames may be acquired both with or without rotation and with or without pullback of the imaging device 308. Moreover, it will be understood that frames may be acquired using other types of movement procedures in addition to, or in lieu of, at least one of rotation or pullback of the imaging device 308.

In some instances, it may desirable to use relatively smaller sized catheters/devices when performing an intravascular imaging procedure. When doing so, it may be desirable to use a rotary motor to rotate a reflector (e.g., rather than a drive cable) to rotate the imaging emitter/transducer. Disclosed herein are imaging device that utilize an imaging core with a rotary motor.

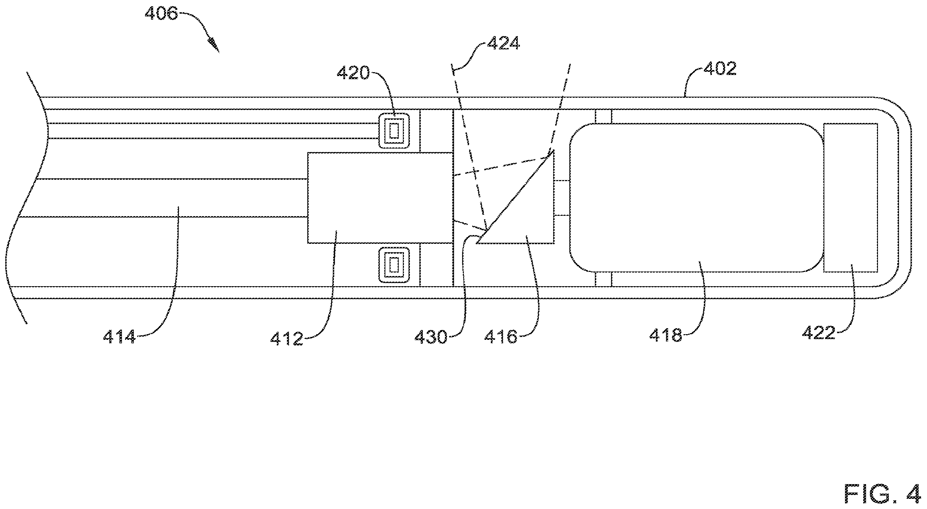

FIG. 4 illustrates another example imaging core 406 that may be similar in form and function to other imaging cores disclosed herein. The imaging core 406 may include a sheath 402. An imaging emitter/transducer 412 may be disposed within the sheath 402. A conductor or transmitter 414 may be coupled to the imaging emitter/transducer 412. For the purposes of this disclosure, the terms "emitter/transducer" and "conductor or transmitter" are meant to encompass a number of different devices and/or components of devices such ultrasound devices (and/or components thereof), ultrasound transducers, optical fibers, light emitting devices, light transmitting devices, lenses, prisms, optical coherence tomography devices (and/or components thereof), conductive members or wires, and/or the like.

A reflector 416 may be disposed within the sheath 402. The reflector 416 may take the form of a mirror. In at least some instances, the reflector 416 may include an angled surface 430. Other reflectors are contemplated including those disclosed herein. Energy 424 (e.g., ultrasonic energy, light energy, etc.) may be transmitted/emitted from the imaging emitter/transducer 412 and be reflected by the reflector 416 toward the sheath 402. The energy 424 may pass through the wall of the sheath 402 (and through the elongate member 202 and/or sheath 302, for example when the imaging core 406 is used with the catheter 102).

A rotary motor 418 may be coupled to the reflector 416. In at least some instances, the rotary motor 418 may take the form of a wireless rotary motor 418 (e.g., a micromotor and/or a wireless micromotor). In such instances, the imaging core 406 may include a transmitter or antenna 420 and a receiver or rectenna 422. In at least some instances, the antenna 420 may be disposed adjacent to the imaging emitter/transducer 412. In at least some instances, the rectenna 422 may be disposed adjacent to the rotary motor 418. In general, the antenna/transmitter 420 may be configured to transmit power to the rectenna/receiver 422 in order to power the rotary motor 418. Thus, the wireless rotary motor 418 may be capable of being powered so as to rotate the reflector 416 without needing to have conductive power wires running between from a power supply to the wireless rotary motor 418. This may be desirable for a number of reasons. For examples, wires extending between the power supply to the rotary motor would extend across the transducer, which could obstruct part of the resultant image. It may be desirable to reduce or avoid obstructions.

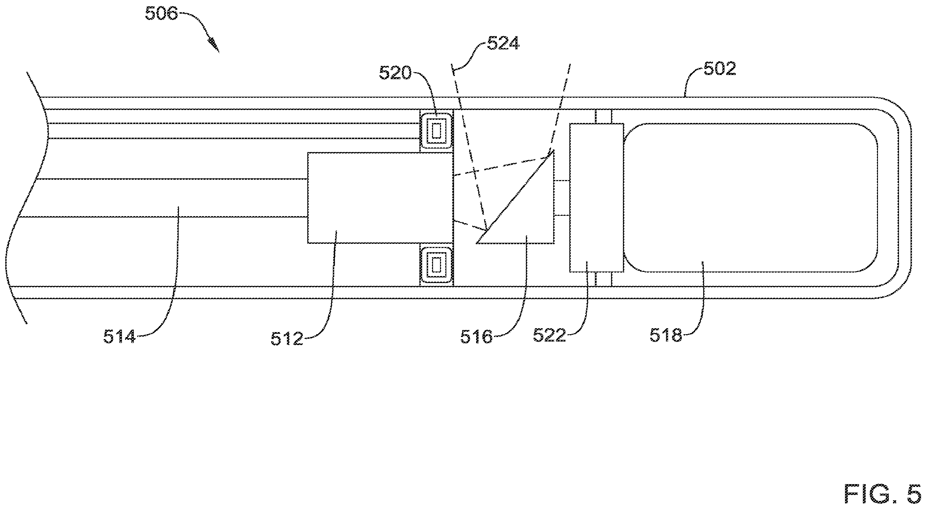

FIG. 5 illustrates another example imaging core 506 that may be similar in form and function to other imaging cores disclosed herein. The imaging core 506 may include a sheath 502. An imaging emitter/transducer 512 may be disposed within the sheath 502. A conductor or transmitter 514 may be coupled to the imaging emitter/transducer 512. A reflector 516 may be disposed within the sheath 502. A wireless rotary motor 518 may be coupled to the reflector 516. Energy 524 may be emitted from the imaging emitter/transducer 512 and be reflected by the reflector 516 toward the sheath 502.

The imaging core 506 may also include a transmitter or antenna 520 and a receiver or rectenna 522. In at least some instances, the antenna 520 may be disposed adjacent to the imaging emitter/transducer 512. In at least some instances, the rectenna 522 may be disposed adjacent to the rotary motor 518. The antenna/transmitter 520 may be configured to transmit power to the rectenna/receiver 522 in order to power the rotary motor 518. In the embodiment depicted in FIG. 4, the rectenna 422 is disposed on a distal end region of the wireless rotary motor 418. This is not intended to be limiting as the location of the various components can vary. For example., in the embodiment depicted in FIG. 5, the rectenna 522 is disposed on a proximal end region of the wireless rotary motor 518. The differing arrangements of the antenna/transmitter 420, 520 and the rectenna/receiver 422, 522 can be used, as appropriate, with any suitable example embodiment such as those disclosed herein.

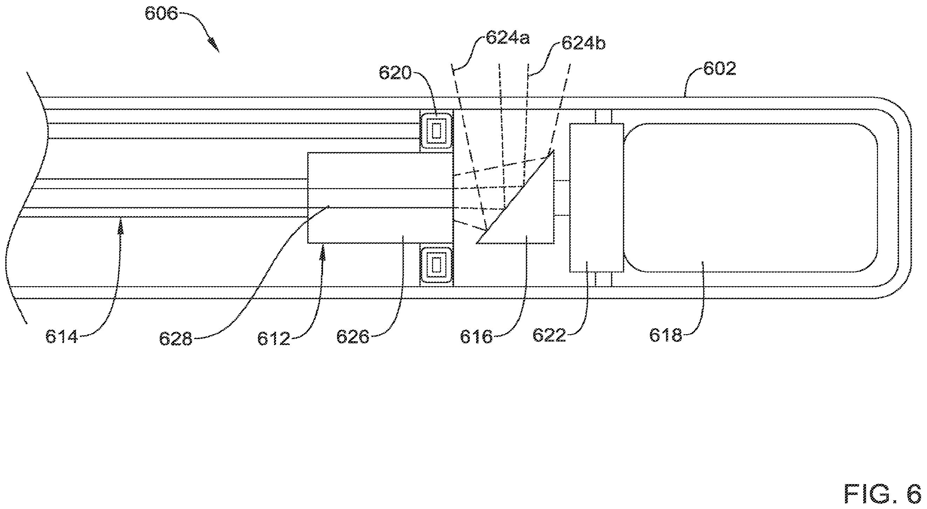

FIG. 6 illustrates another example imaging core 606 that may be similar in form and function to other imaging cores disclosed herein. The imaging core 606 may include a sheath 602. An imaging emitter/transducer 612 may be disposed within the sheath 602. A conductor or transmitter 614 may be coupled to the imaging emitter/transducer 612.

In this example, the imaging emitter/transducer 612 includes a combination device that includes both an ultrasound transducer 626 and an optical fiber 628 (e.g., that can be used an emitter for optical coherence tomography). Thus, the imaging emitter/transducer 612 can be used with an IVUS procedure, an OCT procedure, and/or a combination procedure that utilized both IVUS and OCT. The differing imaging emitter/transducers disclosed herein (e.g., IVUS transducers, ultrasound transducer, OCT devices, optical fiber, etc.) can be used, as appropriate, with any suitable example embodiment such as those disclosed herein.

A reflector 616 may be disposed within the sheath 602. A rotary motor 618 may be coupled to the reflector 616. Energy (e.g., energy 624a from the ultrasound transducer 628 and energy 624b from the optical fiber 628) may be emitted from the imaging emitter/transducer 612 and be reflected by the reflector 616 toward the sheath 602.

The imaging core 606 may also include a transmitter or antenna 620 and a receiver or rectenna 622. In at least some instances, the antenna 620 may be disposed adjacent to the imaging emitter/transducer 612. In at least some instances, the rectenna 622 may be disposed adjacent to the rotary motor 618. The antenna/transmitter 620 may be configured to transmit power to the rectenna/receiver 622 in order to power the rotary motor 618.

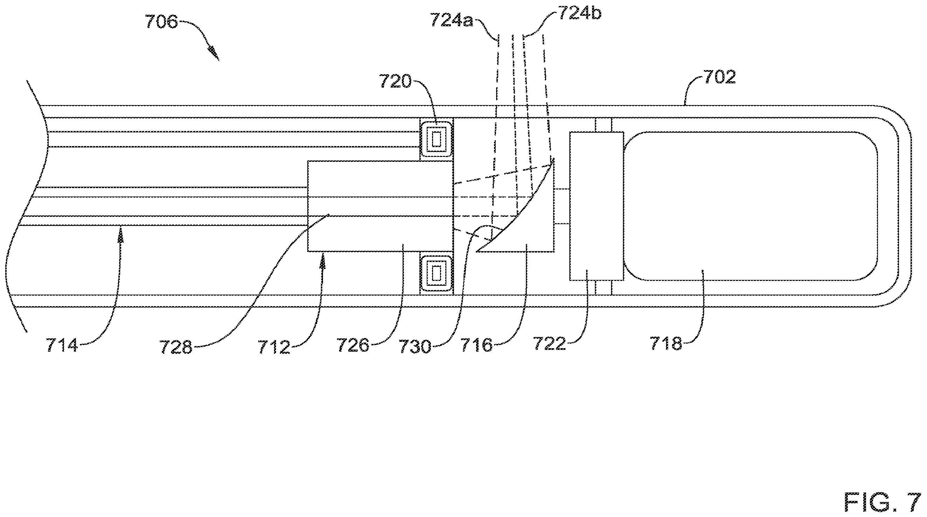

FIG. 7 illustrates another example imaging core 706 that may be similar in form and function to other imaging cores disclosed herein. The imaging core 706 may include a sheath 702. An imaging emitter/transducer 712 may be disposed within the sheath 702. A conductor or transmitter 714 may be coupled to the imaging emitter/transducer 712.

In this example, the imaging emitter/transducer 712 includes both an ultrasound transducer 726 and an optical fiber 728 (e.g., that can be used an emitter for optical coherence tomography).

A reflector 716 may be disposed within the sheath 702. In this example, the reflector 716 may include a curved surface 730. It can be appreciated that the reflector 716 with a curved surface 730 can be used with any suitable example embodiment such as those disclosed herein. A rotary motor 718 may be coupled to the reflector 716. Energy (e.g., energy 724a from the ultrasound transducer 726 and energy 724b from the optical fiber 728) may be emitted from the imaging emitter/transducer 712 and be reflected by the reflector 716 toward the sheath 702.

The imaging core 706 may also include a transmitter or antenna 720 and a receiver or rectenna 722. In at least some instances, the antenna 720 may be disposed adjacent to the imaging emitter/transducer 712. In at least some instances, the rectenna 722 may be disposed adjacent to the rotary motor 718. The antenna/transmitter 720 may be configured to transmit power to the rectenna/receiver 722 in order to power the rotary motor 718.

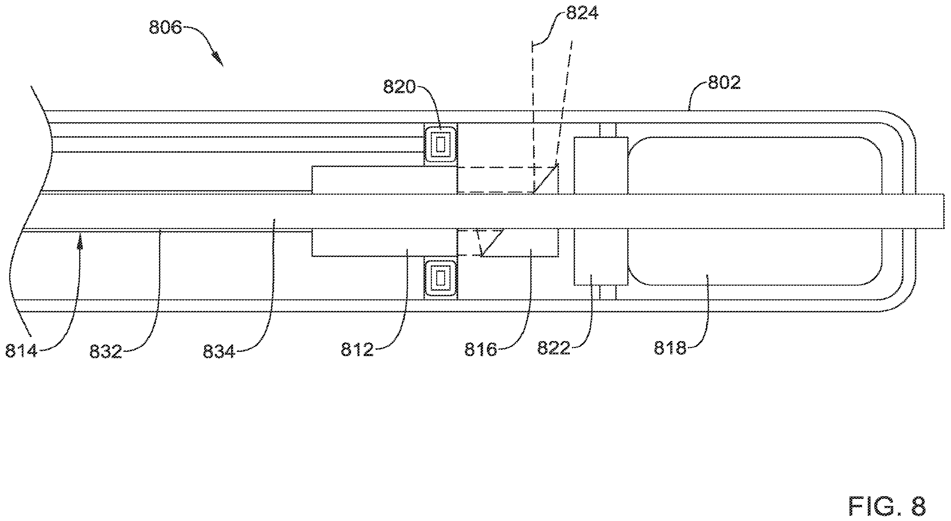

FIG. 8 illustrates another example imaging core 806 that may be similar in form and function to other imaging cores disclosed herein. The imaging core 806 may include a sheath 802. An imaging emitter/transducer 812 may be disposed within the sheath 802. A conductor or transmitter 814 may be coupled to the imaging emitter/transducer 812.

In this example, the conductor 814 may have lumen 832 formed therein. In some instances, the lumen 832 may be a guidewire lumen that is configured to have a guidewire 834 extend therethrough. The guidewire lumen 832 may allow the imaging core 806 (and/or the imaging device) to be tracked over a guidewire 834 in a manner that helps to avoid having the guidewire 834 extend through the imaging field. Thus, obstructions caused by the guidewire 834 can be reduced/eliminated. It can be appreciated that a guidewire lumen such as lumen 832 can be used, where appropriate, with any suitable embodiment disclosed herein.

A reflector 816 may be disposed within the sheath 802. A rotary motor 818 may be coupled to the reflector 816. Energy 824 may be emitted from the imaging emitter/transducer 812 and be reflected by the reflector 816 toward the sheath 802.

The imaging core 806 may also include a transmitter or antenna 820 and a receiver or rectenna 822. In at least some instances, the antenna 820 may be disposed adjacent to the imaging emitter/transducer 812. In at least some instances, the rectenna 822 may be disposed adjacent to the rotary motor 818. The antenna/transmitter 820 may be configured to transmit power to the rectenna/receiver 822 in order to power the rotary motor 818.

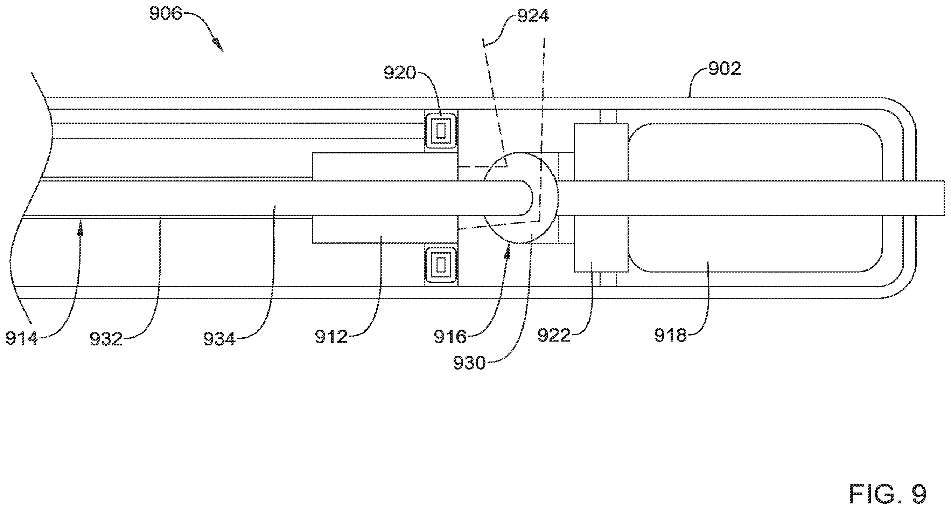

FIG. 9 illustrates another example imaging core 906 that may be similar in form and function to other imaging cores disclosed herein. The imaging core 906 may include a sheath 902. An imaging emitter/transducer 912 may be disposed within the sheath 902. A conductor or transmitter 914 may be coupled to the imaging emitter/transducer 912.

In this example, the conductor 914 may have lumen 932 formed therein. In some instances, the lumen 932 may be a guidewire lumen that is configured to have a guidewire 934 extend therethrough.

A reflector 916 may be disposed within the sheath 902. In this example, the reflector 916 may include an annular reflective surface 930. It can be appreciated that the reflector 916 with the annular reflective surface 930 can be used with any suitable example embodiment such as those disclosed herein. A rotary motor 918 may be coupled to the reflector 916. Energy 924 may be emitted from the imaging emitter/transducer 912 and be reflected by the reflector 916 toward the sheath 902.

The imaging core 906 may also include a transmitter or antenna 920 and a receiver or rectenna 922. In at least some instances, the antenna 920 may be disposed adjacent to the imaging emitter/transducer 912. In at least some instances, the rectenna 922 may be disposed adjacent to the rotary motor 918. The antenna/transmitter 920 may be configured to transmit power to the rectenna/receiver 922 in order to power the rotary motor 918.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. An intravascular imaging device, comprising:
a catheter shaft;
an imaging core (406, 506, 606, 706, 806, 906) disposed within the catheter shaft, the imaging core including a sheath (402, 502, 602, 702, 802, 902), an imaging emitter/transducer (412, 512, 612, 712, 812, 912) secured relative to the sheath, a reflector (416, 516, 616, 716, 816, 916), and a wireless rotary motor (418, 518, 618, 718, 818, 918) coupled to the reflector;
wherein the wireless rotary motor is configured to rotate the reflector relative to the imaging emitter/transducer;
**characterised in that** the intravascular device further comprises
an antenna (420, 520, 620, 720, 820, 920) disposed adjacent to the imaging emitter/transducer; and
a rectenna (422, 522, 622, 722, 822, 922) disposed adjacent to the wireless rotary motor;
wherein the antenna is configured to transmit power to the rectenna in order to power the wireless rotary motor.

2. The intravascular imaging device of claim 1, wherein the imaging emitter/transducer includes an ultrasound transducer.

3. The intravascular imaging device of any one of claims 1-2, wherein the imaging emitter/transducer includes an optical coherence tomography emitter.

4. The intravascular imaging device of any one of claims 1-3, wherein the imaging emitter/transducer includes both an ultrasound transducer and an optical coherence tomography emitter.

5. The intravascular imaging device of any one of claims 1-4, wherein the rectenna is disposed adjacent to a distal end region of the wireless rotary motor.

6. The intravascular imaging device of any one of claims 1-4, wherein the rectenna is disposed adjacent to a proximal end region of the wireless rotary motor.

7. The intravascular imaging device of any one of claims 1-6, wherein the reflector includes an angled surface.

8. The intravascular imaging device of any one of claims 1-7, wherein the reflector includes a curved surface.

9. The intravascular imaging device of any one of claims 1-8, wherein the reflector includes an annular surface.

10. The intravascular imaging device of any one of claims 1-9, wherein a guidewire lumen is formed in the imaging core.

11. The intravascular imaging device of claim 10, wherein the guidewire lumen extends through the imaging emitter/transducer, the wireless rotary motor, or both.

12. The intravascular imaging device of claim 1,
wherein the imaging emitter/transducer includes an ultrasound transducer, and the ultrasound transducer is rotationally fixed relative to the sheath.

13. The intravascular imaging device of claim 12, wherein a guidewire lumen is formed in the imaging core; and
wherein the guidewire lumen extends through the imaging emitter/transducer, the wireless rotary motor, or both.

## Patentansprüche

1. Intravaskuläre Bildgebungsvorrichtung, aufweisend:
einen Katheterschaft;
einen Bildgebungskern (406, 506, 606, 706, 806, 906) der innerhalb des Katheterschafts angeordnet ist, wobei der Bildgebungskern eine Hülle (402, 502, 602, 702, 802, 902), einen Bildgebungsemitter/-wandler (412, 512, 612, 712, 812, 912), der relativ zur Hülle gesichert ist, einen Reflektor (416, 516, 616, 716, 816, 916) und einen mit dem Reflektor verbundenen drahtlosen Drehmotor (418, 518, 618, 718, 818, 918) aufweist, wobei der drahtlose Drehmotor dafür konfiguriert ist, den Reflektor relativ zum Bildgebungsemitter/-wandler zu drehen;
**dadurch gekennzeichnet, dass**
die intravaskuläre Vorrichtung ferner aufweist:
eine Antenne (420, 520, 620, 720, 820, 920) die benachbart zu dem Bildgebungsemitter/-wandler angeordnet ist; und
eine Rectenna (422, 522, 622, 722, 822, 922) die benachbart zu dem drahtlosen Drehmotor angeordnet ist,
wobei die Antenne dafür konfiguriert ist, Energie an die Rectenna zu übertragen, um den drahtlosen Drehmotor anzutreiben.

2. Intravaskuläre Bildgebungsvorrichtung nach Anspruch 1, wobei der Bildgebungsemitter/-wandler einen Ultraschallwandler aufweist.

3. Intravaskuläre Bildgebungsvorrichtung nach Anspruch 1 oder 2, wobei der Bildgebungsemitter/-wandler einen Emitter für optische Kohärenztomographie aufweist.

4. Intravaskuläre Bildgebungsvorrichtung nach einem der Ansprüche 1 bis 3, wobei der Bildgebungsemitter /-wandler sowohl einen Ultraschallwandler als auch einen Emitter für optische Kohärenztomographie aufweist.

5. Intravaskuläre Bildgebungsvorrichtung nach einem der Ansprüche 1 bis 4, wobei die Rectenna benachbart zu einem distalen Endbereich des drahtlosen Drehmotors angeordnet ist.

6. Intravaskuläre Bildgebungsvorrichtung nach einem der Ansprüche 1 bis 4, wobei die Rectenna benachbart zu einem proximalen Endbereich des drahtlosen Drehmotors angeordnet ist.

7. Intravaskuläre Bildgebungsvorrichtung nach einem der Ansprüche 1 bis 6, wobei der Reflektor eine abgewinkelte Oberfläche aufweist.

8. Intravaskuläre Bildgebungsvorrichtung nach einem der Ansprüche 1 bis 7, wobei der Reflektor eine gekrümmte Oberfläche aufweist.

9. Intravaskuläre Bildgebungsvorrichtung nach einem der Ansprüche 1 bis 8, wobei der Reflektor eine ringförmige Oberfläche aufweist.

10. Intravaskuläre Bildgebungsvorrichtung nach einem der Ansprüche 1 bis 9, wobei ein Führungsdrahtlumen im Bildgebungskern ausgebildet ist.

11. Intravaskuläre Bildgebungsvorrichtung nach Anspruch 10, wobei sich das Führungsdrahtlumen durch den Bildgebungsemitter/-wandler und/oder den drahtlosen Drehmotor erstreckt.

12. Intravaskuläre Bildgebungsvorrichtung nach Anspruch 1,
wobei der Bildgebungsemitter/-wandler einen Ultraschallwandler aufweist und der Ultraschallwandler relativ zur Hülle drehfest angeordnet ist.

13. Intravaskuläre Bildgebungsvorrichtung nach Anspruch 12, wobei ein Führungsdrahtlumen im Bildgebungskern ausgebildet ist, und
wobei sich das Führungsdrahtlumen durch den Bildgebungsemitter/- wandler und/oder den drahtlosen Drehmotor erstreckt.

## Revendications

1. Dispositif d'imagerie intravasculaire, comprenant:
une tige de cathéter;
un noyau d'imagerie (406, 506, 606, 706, 806, 906) disposé à l'intérieur de la tige de cathéter, le noyau d'imagerie incluant une gaine (402, 502, 602, 702, 802, 902), un émetteur/transducteur d'imagerie (412, 512, 612, 712, 812, 912) fixé par rapport à la gaine, un réflecteur (416, 516, 616, 716, 816, 916) et un moteur rotatif sans fil (418, 518, 618, 718, 818, 918) couplé au réflecteur;
dans lequel le moteur rotatif sans fil est configuré pour faire tourner le réflecteur par rapport à l'émetteur/transducteur d'imagerie;
**caractérisé en ce que** le dispositif intravasculaire comprend en outre
une antenne (420, 520, 620, 720, 820, 920) disposée de manière adjacente à l'émetteur/transducteur d'imagerie; et
une antenne redresseuse (422, 522, 622, 722, 822, 922) disposée de manière adjacente au moteur rotatif sans fil;
dans lequel l'antenne est configurée pour transmettre de l'énergie à l'antenne redresseuse afin d'alimenter le moteur rotatif sans fil.

2. Dispositif d'imagerie intravasculaire selon la revendication 1, dans lequel l'émetteur/transducteur d'imagerie inclut un transducteur à ultrasons.

3. Dispositif d'imagerie intravasculaire selon l'une quelconque des revendications 1 et 2, dans lequel l'émetteur/transducteur d'imagerie inclut un émetteur de tomographie par cohérence optique.

4. Dispositif d'imagerie intravasculaire selon l'une quelconque des revendications 1 à 3, dans lequel l'émetteur/transducteur d'imagerie inclut à la fois un transducteur à ultrasons et un émetteur de tomographie par cohérence optique.

5. Dispositif d'imagerie intravasculaire selon l'une quelconque des revendications 1 à 4, dans lequel l'antenne redresseuse est disposée de manière adjacente à une région d'extrémité distale du moteur rotatif sans fil.

6. Dispositif d'imagerie intravasculaire selon l'une quelconque des revendications 1 à 4, dans lequel l'antenne redresseuse est disposée de manière adjacente à une région d'extrémité proximale du moteur rotatif sans fil.

7. Dispositif d'imagerie intravasculaire selon l'une quelconque des revendications 1 à 6, dans lequel le réflecteur inclut une surface inclinée.

8. Dispositif d'imagerie intravasculaire selon l'une quelconque des revendications 1 à 7, dans lequel le réflecteur inclut une surface incurvée.

9. Dispositif d'imagerie intravasculaire selon l'une quelconque des revendications 1 à 8, dans lequel le réflecteur inclut une surface annulaire.

10. Dispositif d'imagerie intravasculaire selon l'une quelconque des revendications 1 à 9, dans lequel une lumière de fil guide est formée dans le noyau d'imagerie.

11. Dispositif d'imagerie intravasculaire selon la revendication 10, dans lequel la lumière de fil guide s'étend à travers l'émetteur/transducteur d'imagerie, le moteur rotatif sans fil, ou les deux.

12. Dispositif d'imagerie intravasculaire selon la revendication 1, dans lequel l'émetteur/transducteur d'imagerie inclut un transducteur à ultrasons, et le transducteur à ultrasons est fixé en rotation par rapport à la gaine.

13. Dispositif d'imagerie intravasculaire selon la revendication 12, dans lequel une lumière de fil guide est formée dans le noyau d'imagerie; et
dans lequel la lumière de fil guide s'étend à travers l'émetteur/transducteur d'imagerie, le moteur rotatif sans fil, ou les deux.
